(19) Europäisches Patentamt
European Patent Office
Office européen des brevets

(11) Publication number: **0 333 351 B1**

# (12) EUROPEAN PATENT SPECIFICATION

(45) Date of publication of patent specification: **15.02.95**

(51) Int. Cl.[6]: **C07F 15/00**, A61K 31/28

(21) Application number: **89302111.3**

(22) Date of filing: **02.03.89**

## (54) Platinum coordination compounds.

(30) Priority: **14.03.88 GB 8806044**

(43) Date of publication of application:
**20.09.89 Bulletin 89/38**

(45) Publication of the grant of the patent:
**15.02.95 Bulletin 95/07**

(84) Designated Contracting States:
**AT BE CH DE ES FR GB GR IT LI LU NL SE**

(56) References cited:
**EP-A- 0 147 926**
**CH-A- 633 961**

(73) Proprietor: **JOHNSON MATTHEY PUBLIC LIMITED COMPANY**
**78 Hatton Garden**
**London EC1N 8JP (GB)**

(72) Inventor: **Barnard, Christopher Francis James**
**3 Lady Jane Court**
**Grosvenor Road**
**Caversham**
**Reading RG4 OEH (GB)**
Inventor: **Hydes, Paul Cedric**
**28 Bolaro Avenue**
**Gymea, NSW 2227 (AU)**

(74) Representative: **Harrison, Ivor Stanley et al**
**Withers & Rogers**
**4 Dyer's Buildings**
**Holborn**
**London EC1N 2JT (GB)**

**EP 0 333 351 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention concerns platinum coordination compounds indicated for chemotherapeutic use. More especially, it concerns platinum (IV) coordination compounds.

Certain platinum coordination compounds have become accepted for chemotherapeutic use against a variety of tumours. The best-known compound is "cisplatin", cis-diammine-dichloro-platinum (II), which has activity against a broad spectrum of tumours. The toxicity of cisplatin, however, leads to unpleasant side-effects or possibly the rejection by the patient of chemotherapy. A further platinum (II) compound, carboplatin, has recently been introduced, and which exhibits considerably less toxicity. There remains, however, a need for chemotherapeutic agents which are of relatively low toxicity and/or are active against tumours which are unaffected by or resistant to existing platinum chemotherapeutic agents.

CH-A 633961 and related GB-A 1,585,103 disclose Pt(IV) coordination compounds of a broad class, and the disclosures thereof are incorporated herein by reference. In these specifications, bis-n-alkylamine Pt(IV) compounds alone were exemplified and the preparative method disclosed was only suitable for the preparation of bis-alkylamine compounds.

USP 4,329,299, discloses mixed amine Pt (II) and Pt (IV) compounds as chemotherapeutic agents. Only Pt (II) compounds were exemplified and tested. European Specification EP 0 147 926A is directed to the use of platinum (II) and (IV) compounds for oral treatment; only one Pt (IV) compound is mentioned cis-amminetetrachloro (cyclohexylamine)-platinum(IV) which was stated to be known per se.

We have now discovered that certain platinum (IV) coordination compounds have unexpected activity in in vitro cytotoxicity tests, especially against cisplatin-resistant and Tetraplatin-resistant tumour cells, which indicates potential clinical utility. Accordingly, the present invention provides a platinum (IV) coordination compound of the general formula I,

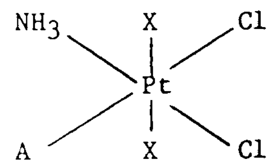

(I)

in which X is a chlorine atom or hydroxyl group, and

A is an amine of the general formula $R-NH_2$ wherein

R is a straight chain alkyl of 2 or 3 carbon atoms, alicyclic of 3 to 7 carbon atoms, or branched chain alkyl of 3 or 4 carbon atoms, provided that where X is a chlorine atom R is not cyclohexyl. Preferred compounds are those of formula Ia,

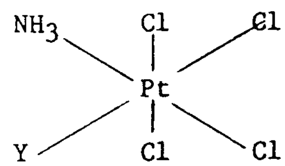

(Ia)

in which Y is an amine of the formula $R'-NH_2$ wherein

R' is branched chain alkyl of 3 or 4 carbon atoms or alicyclic of 3 to 5 or 7 carbon atoms.

Other preferred compounds are those of formula Ib,

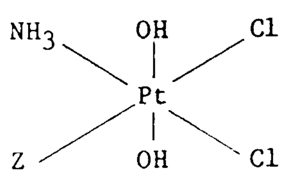

(Ib)

in which Z is an amine of the formula R''-$NH_2$ wherein

R'' is branched chain alkyl of 3 or 4 carbon atoms or alicyclic of 3 to 7 carbon atoms.

Especially preferred compounds are those of formula Ia or Ib wherein R' or R'' respectively are alicyclic or isopropyl.

The compounds of formula I, Ia and Ib are believed to be novel, although falling within prior generic disclosures. They may suitably be prepared from the initial starting material potassium amminetrichloroplatinate (II). The invention provides a method for the preparation of compounds of the formula I, comprising (a) reacting a platinum (II) complex of the general formula II,

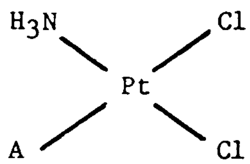

(II)

in which A is as defined above,

with chlorine to form a compound of formula I as defined above but in which X is a chlorine atom, and (b) where a compound of formula I in which X is a hydroxyl group is desired, treating the dichloro compound of formula II with hydrogen peroxide.

The compound of formula II may be prepared by converting the starting material $K[PtCl_3(NH_3)]$ into the appropriate alkylamine ammine diiodo complex by solution in water, and adding potassium iodide and the appropriate alkylamine. The diiodo complex is then treated with silver nitrate and stirred for 2 hours in the dark, charcoal is added and the mixture filtered. The filtrate is tested for free silver ion and, if necessary, sodium chloride solution added until silver ion cannot be detected. The filtrate is then added to excess concentrated hydrochloric acid and stirred, and the compound of formula II is collected by filtration.

Step (a) of the method may be suitably carried out by suspending the compound of formula II in water, and passing chlorine gas through the suspension, conveniently for 1 hour at a reaction temperature of 60°C. The reaction mixture may then be boiled to remove excess chlorine, and cooled, and the tetrachloro complex collected by filtration, washed and dried in vacuo.

Step (b) of the method may be suitably carried out by suspending the dichloro complex of formula II in water, adding hydrogen peroxide, eg 30% w/v, and stirring, conveniently at 60°C for two hours. After cooling, the product may be collected by filtration, washed with water and recrystallised from water.

The present invention further provides a pharmaceutical composition comprising a compound of formula I, Ia or Ib, in association with a pharmaceutically acceptable diluent or carrier. The invention additionally provides a pharmaceutical composition in unit dosage form, comprising an effective unit dose of a compound of formula I, Ia or Ib, in association with a pharmaceutically acceptable carrier or diluent. Preferred unit doses may be in the range 10mg to 1g of active compound. If desired, other pharmaceutically active compounds may be combined with the compound of formula I, Ia or Ib.

Pharmaceutical compositions and unit doses may be prepared by methods and in forms generally known in the pharmaceutical art. Suitable carriers or diluents are well known, and additional components such as binders, excipients, lubricants, flavouring agents etc may be used depending on the desired dosage form, eg tablets, capsules, syrups or suspensions for oral administration, or solutions for intraperitoneal administration.

The invention will now be described by way of example only. A selection of compounds according to the invention were prepared by the above-described method, and were characterised by elemental analysis and infra red spectroscopy. The compounds were compared against known compounds and against each other for activity against the ADJ/PC6 tumour in mice, when given by the intraperitoneal route and the results are tabulated below.

TABLE 1

| COMPOUND | ANTITUMOUR ACTIVITY ADJ/PC6 (mg/kg) | | |
|---|---|---|---|
| | $LD_{50}$ | $ED_{90}$ | T.I. |
| (Comparison A) 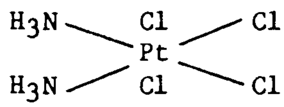 | 18 | 3.4 | 5 |
| (Comparison B) 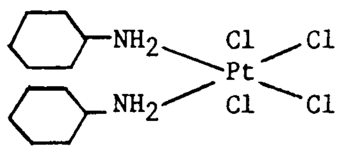 | 190 | 10 | 20 |
| 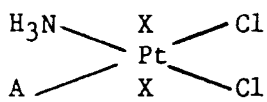 (I) | | | |
| X = Cl A = $(CH_3)_2$ $CHCNH_2$ | 9 | 0.4 | 23 |
| X = Cl A = 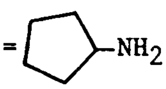 | 18 | 0.8 | 22 |
| X = Cl A = $(CH_3)_2CHCH_2CH_2NH_2$ | 35 | 5.5 | 6 |
| X = Cl A = $(CH_3)_3CNH_2$ | 15 | 0.8 | 19 |
| X = Cl A = $CH_3CH_2$ $C(CH_3)_2NH_2$ | 35 | 8.0 | 4 |

Note: Although comparison B exhibited a low toxicity, it was essentially insoluble and could not readily be administered.

TABLE 2

| 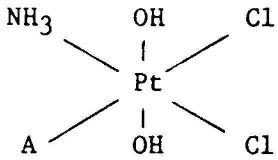 | ANTITUMOUR ACTIVITY ADJ/PC6 i.p. (mg/kg) | | |
|---|---|---|---|
| A | $LD_{50}$ | $ED_{90}$ | T.I. |
| $NH_3$ (Comparison C) | 135 | 5 | 28 |
| 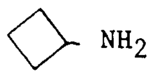 | 42 | 3 | 14 |
| 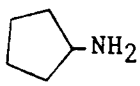 | 21 | 0.9 | 23 |
| 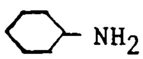 | 17 | 0.4 | 41 |
| 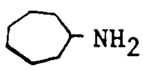 | 21 | 1.4 | 15 |
| 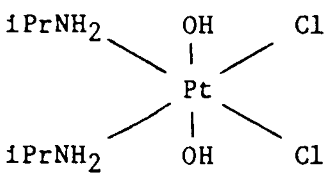 (Comparison D) | 90 | 7.5 | 12 |
| $iPrNH_2$ | 35 | 3.0 | 12 |
| $(CH_3)_2CHCH_2CH_2NH_2$ | 35 | 7.2 | 5 |
| $(CH_3)_3CNH_2$ | 36 | 4.9 | 7 |
| $CH_3CH_2C(CH_3)_2NH_2$ | 21 | 5.7 | 4 |
| $(CH_3)_3CCH_2C(CH_3)_2NH_2$ | 82 | 20 | 4 |

Several of the compounds according to the invention were also tested in vitro for activity against L1210 leukaemia cell lines and against L1210 cell lines which had been bred to be resistant to cisplatin and Tetraplatin. The results are tabulated below:

TABLE 3

| 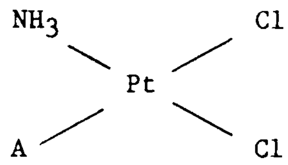 | L1210 $IC_{50}$ ($^{e}$M) | | |
|---|---|---|---|
| A | L1210 | Cisplatin Resistant | Tetraplatin Resistant |
| $(CH_3)_2CHCH_2NH_2$ | 0.2 | 0.3 | 2.7 |
| $(CH_3)_3CNH_2$ | 0.8 | 1.2 | 7.1 |
| (comparisons) | | | |

| 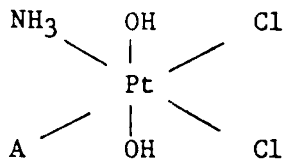 | L1210 $IC_{50}$ ($^{e}$M) | | |
|---|---|---|---|
| A | L1210 | Cisplatin Resistant | Tetraplatin Resistant |
| cyclo-$C_4H_7NH_2$ | 4.4 | 5.1 | 5.1 |
| cyclo-$C_5H_9NH_2$ | 1.4 | 1.7 | 2.3 |
| cyclo-$C_6H_{11}NH_2$ | 0.6 | 0.7 | 0.4 |
| cyclo-$C_7H_{13}NH_2$ | 0.4 | 0.7 | 0.5 |
| i-$C_3H_7NH_2$ | 8.5 | 8.8 | 11.5 |
| i-$C_4H_9NH_2$ | 5.9 | 5.1 | 9.0 |

The $IC_{50}$ values are defined as the concentration of each compound required to reduce cell counts to 50% of control values after 48 hours continuous exposure.

It can readily be seen that the hydroxy components according to the invention are surprisingly effective against cells which are resistant to treatment by other platinum coordination compounds.

In addition the following compound has been prepared and characterised:

$PtCl_4(NH_3)(c\text{-}C_3H_5\ NH_2)$

## Claims

**Claims for the following Contracting States : AT, BE, CH, DE, FR, GB, IT, LI, LU, NL, SE**

1. A platinum (IV) coordination compound of the general formula I,

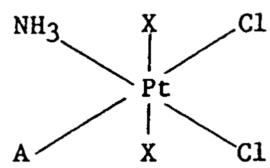

(I)

in which X is a chlorine atom or hydroxyl group, and

A is an amine of the general formula $R\text{-}NH_2$ wherein

R is straight chain alkyl of 2 or 3 carbon atoms, alicyclic of 3 to 7 carbon atoms or branched chain alkyl of 3 or 4 carbon atoms, provided that where X is a chlorine atom R is not cyclohexyl.

2. A compound according to claim 1, wherein X is chlorine and A is a branched chain alkylamine of 3 or 4 carbon atoms or an alicyclic amine of 3 to 5 or 7 carbon atoms.

3. A compound according to claim 1, wherein X is hydroxyl and A is a branched chain alkylamine of 3 or 4 carbon atoms or an alicyclic amine of 3 to 7 carbon atoms.

4. A pharmaceutical composition comprising a platinum compound according to any one of claims 1 to 3, in association with a pharmaceutically acceptable carrier or diluent.

5. A pharmaceutical composition comprising a platinum compound according to any one of claims 1 to 3, in unit dosage form, comprising an effective unit dose of the platinum compound in association with a pharmaceutically acceptable carrier or diluent.

6. A pharmaceutical composition according to claim 4, for the treatment of tumours resistant to treatment by platinum coordination compounds of different structure.

7. A process for the production of a compound according to any one of claims 1 to 3, comprising (a) reacting a platinum (II) complex of the general formula II,

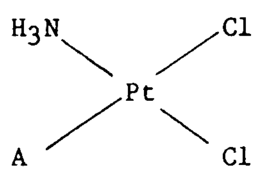

(II)

in which A is as defined in claim 1,
with chlorine to form a compound of formula I as defined in claim 1 but in which X is a chlorine atom, and (b) where a compound of formula I in which X is a hydroxyl group is desired, treating the dichloro compound of formula II with hydrogen peroxide.

**Claims for the following Contracting State : ES**

**1.** A process for the production of a platinum (IV) coordination compound of the general formula I,

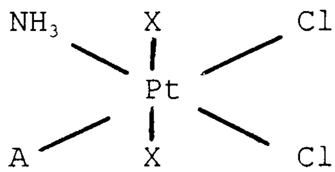

(I)

in which X is a chlorine atom or hydroxyl group, and

A is an amine of the general formula $R-NH_2$ wherein R is a straight chain alkyl of 2 or 3 carbon atoms, alicyclic of 3 to 7 carbon atoms or branched chain alkyl of 3 or 4 carbon atoms, provided that where X is a chlorine atom R is not cyclohexyl, the process comprising reacting a platinum (II) complex of the general formula II,

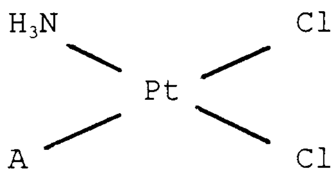

(II)

in which A is as defined above, either with chlorine to form a compound of formula I in which X is a chlorine atom, or with hydrogen peroxide to form a compound of formula I in which X is a hydroxyl group.

**2.** A process according to Claim 1 in which the complex (II) is reacted with chlorine, wherein A is a branched chain alkylamine of 3 or 4 carbon atoms or an alicyclic amine of 3 to 5 or 7 carbon atoms.

**3.** A process according to Claim 1 in which the complex (II) is reacted with hydrogen peroxide, wherein A is a branched chain alkylamine of 3 or 4 carbon atoms or an alicyclic amine of 3 to 7 carbon atoms.

**4.** A process according to Claim 1 or Claim 2, in which the complex (II) is suspended in water and chlorine gas is passed through the suspension, the reaction mixture boiled if necessary to remove excess chlorine, and the tetrachloro complex is collected by filtration.

**5.** A process according to Claim 1 or Claim 3, in which the complex (II) is suspended in water, hydrogen peroxide solution is added to the suspension with stirring and the product is collected by filtration.

**6.** A process according to any preceding claim, in which the complex (II) is prepared by converting $K-[PtCl_3(NH_3)]$ into the appropriate alkylamine ammine diiodo complex by dissolution in water and addition of potassium iodide and the appropriate alkylamine, treating the diiodo complex with silver nitrate and adding excess concentrated hydrochloric acid.

**7.** A process for the preparation of a pharmaceutical composition, the process comprising addition of a compound of formula (I) as defined in Claim 1 when prepared by a process according to any preceding claim to a pharmaceutically acceptable carrier or diluent.

**Claims for the following Contracting State : GR**

**1.** A platinum (IV) coordination compound of the general formula I,

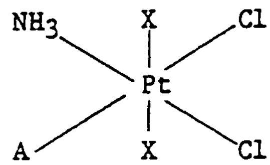

(I)

in which X is a chlorine atom or hydroxyl group, and
A is an amine of the general formula R-$NH_2$ wherein
R is straight chain alkyl of 2 or 3 carbon atoms, alicyclic of 3 to 7 carbon atoms or branched chain alkyl of 3 or 4 carbon atoms, provided that where X is a chlorine atom R is not cyclohexyl.

**2.** A compound according to claim 1, wherein X is chlorine and A is a branched chain alkylamine of 3 or 4 carbon atoms or an alicyclic amine of 3 to 5 or 7 carbon atoms.

**3.** A compound according to claim 1, wherein X is hydroxyl and A is a branched chain alkylamine of 3 or 4 carbon atoms or an alicyclic amine of 3 to 7 carbon atoms.

**4.** A process for the production of a compound according to any one of Claims 1 to 3, comprising reacting a platinum (II) complex of the general formula II,

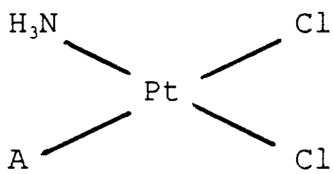

(II)

in which A is defined in Claim 1, either with chlorine to form a compound of formula I as defined in Claim 1 in which X is a chlorine atom, or with hydrogen peroxide to form a compound of formula I in which X is a hydroxyl group.

**5.** A process according to Claim 4, in which the complex (II) is suspended in water and chlorine gas is passed through the suspension, the reaction mixture boiled if necessary to remove excess chlorine, and the tetrachloro complex is collected by filtration.

**6.** A process according to Claim 4, in which the complex (II) is suspended in water, hydrogen peroxide solution is added to the suspension with stirring and the product is collected by filtration.

**7.** A process according to any preceding claim, in which the complex (II) is prepared by converting K-[$PtCl_3(NH_3)$] into the appropriate alkylamine ammine diiodo complex by dissolution in water and addition of potassium iodide and the appropriate alkylamine, treating the diiodo complex with silver nitrate and adding excess concentrated hydrochloric acid.

**8.** A process for the preparation of a pharmaceutical composition, the process comprising addition of a compound of formula (I) as defined in Claim 1 to a pharmaceutically acceptable carrier or diluent.

**Patentansprüche**
**Patentansprüche für folgende Vertragsstaaten : AT, BE, CH, DE, FR, GB, IT, LI, LU, NL, SE**

1. Platin(IV)-Koordinationsverbindung der allgemeinen Formel I,

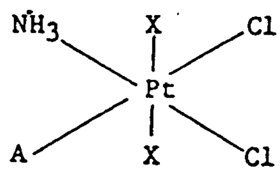

(I)

wobei
X ein Chlor-Atom oder eine Hydroxyl-Gruppe, und
A ein Amin der allgemeinen Formel $R\text{-}NH_2$ sind,
wobei
R eine geradkettige Alkyl-Kette aus 2 oder 3 Kohlenstoff-Atomen, eine alicyclische Gruppe aus 3 - 7 Kohlenstoff-Atomen oder eine verzweigte Alkyl-Kette aus 3 oder 4 Kohlenstoff-Atomen ist, mit der Maßgabe, daß R keine Cyclohexyl-Gruppe ist, wenn X Chlor ist.

2. Die Verbindung nach Anspruch 1, wobei X Chlor und A ein Amin einer verzweigten Alkyl-Kette mit 3 oder 4 Kohlenstoff-Atomen oder ein Amin einer alicyclischen Gruppe mit 3 - 5 oder 7 Kohlenstoff-Atomen sind.

3. Die Verbindung nach Anspruch 1, wobei X Hydroxyl und A ein Amin einer verzweigten Alkyl-Kette mit 3 oder 4 Kohlenstoff-Atomen oder ein Amin einer alicyclischen Gruppe mit 3 - 7 Kohlenstoff-Atomen sind.

4. Pharmazeutische Zusammensetzung, umfassend eine Platin-Verbindung gemaß einem der Ansprüche 1 - 3, in Verbindung mit einem pharmazeutisch verträglichen Träger oder Verdünnungsmittel.

5. Pharmazeutische Zusammensetzung, umfassend eine Platin-Verbindung gemäß einem der Ansprüche 1 - 3, in Einheitsdosierungsform, umfassend eine wirksame Einheitsdosis der Platin-Verbindung in Verbindung mit einem pharmazeutisch verträglichen Träger oder Verdünnungsmittel.

6. Die pharmazeutische Zusammensetzung nach Anspruch 4 für die Behandlung von Tumoren, die gegenüber der Behandlung mit Platin-Kooridinationsverbindungen anderer Struktur resistent sind.

7. Verfahren zur Herstellung einer Verbindung gemäß einem der Ansprüche 1 - 3, wobei das Verfahren den Schritt umfaßt, (a) einen Platin(II)-Komplex der allgemeinen Formel II

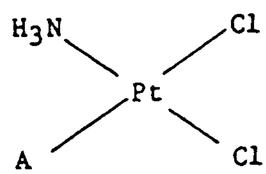

(II)

wobei A wie in Anspruch 1 definiert ist, unter Bildung einer Verbindung der Formel I, wie sie in Anspruch 1 definiert ist, wobei X aber ein Chlor-Atom ist, mit Chlor und (b) die Dichlor-Verbindung der Formel II mit Wasserstoffperoxid zu behandeln, falls eine Verbindung der Formel I erwünscht ist, in der X eine Hydroxyl-Gruppe ist.

**Patentansprüche für folgenden Vertragsstaat : ES**

1. Verfahren zur Herstellung einer Platin(IV)-Koordinationsverbindung der allgemeinen Formel I,

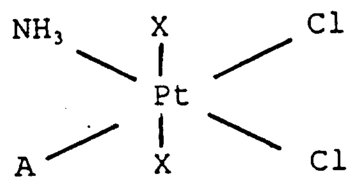

(I)

wobei

X ein Chlor-Atom oder eine Hydroxyl-Gruppe, und

A ein Amin der allgemeinen Formel R-$NH_2$ sind,

wobei

R eine geradkettige Alkyl-Kette aus 2 oder 3 Kohlenstoff-Atomen, eine alicyclische Gruppe aus 3 - 7 Kohlenstoff-Atomen oder eine verzweigte Alkyl-Kette aus 3 oder 4 Kohlenstoff-Atomen ist, mit der Maßgabe, daß R keine Cyclohexyl-Gruppe ist, wenn X Chlor ist,

wobei das Verfahren die Schritte umfaßt, einen Platin (II) - Komplex der allgemeinen Formel II

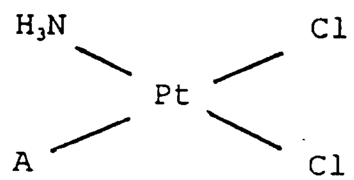

(II)

wobei A wie oben definiert ist, entweder unter Bildung einer Verbindung der Formel I, wobei X ein Chlor-Atom ist, mit Chlor oder unter Bildung einer Verbindung der Formel I, bei der X eine Hydroxyl-Gruppe ist, mit Wasserstoffperoxid umzusetzen.

2. Das Verfahren nach Anspruch 1, wobei der Komplex (II) mit Chlor umgesetzt wird, wobei A ein Amin einer verzweigten Alkyl-Kette mit 3 oder 4 Kohlenstoff-Atomen oder ein Amin einer alicyclischen Gruppe mit 3 - 5 oder 7 Kohlenstoff-Atomen ist.

3. Das Verfahren nach Anspruch 1, wobei der Komplex (II) mit Wasserstoffperoxid umgesetzt wird, wobei A ein Amin einer verzweigten Alkyl-Kette mit 3 oder 4 Kohlenstoff-Atomen oder ein Amin einer alicyclischen Gruppe mit 3 - 7 Kohlenstoff-Atomen ist.

4. Das Verfahren nach Anspruch 1 oder 2, wobei der Komplex (II) in Wasser suspendiert wird, und Chlorgas durch die Suspension geleitet wird, das Reaktionsgemisch zur Entfernung überschüssigen Chlors, falls nötig, gekocht wird, und der Tetrachlor-Komplex mittels Filtration gesammelt wird.

5. Das Verfahren nach Anspruch 1 oder 3, wobei der Komplex (II) in Wasser suspendiert wird, eine Wasserstoffperoxid-Lösung der Suspension unter Rühren zugesetzt wird, und das Produkt mittels Filtration gesammelt wird.

6. Das Verfahren nach einem der vorhergehenden Ansprüche, wobei der Komplex (II) dadurch hergestellt wird, daß $K[PtCl_3(NH_3)]$ durch Lösen in Wasser und Zugabe von Kaliumiodid und dem geeigneten Alkylamin in den geeigneten Alkylamin-Amin-Diiod-Komplex umgewandelt, der Diiod-Komplex mit Silbernitrat behandelt und konzentrierte Salzsäure im Überfluß zugesetzt wird.

7. Verfahren zur Herstellung einer pharmazeutischen Zusammensetzung, wobei das Verfahren die Zugabe einer Verbindung der Formel (I), wie sie in Anspruch 1 definiert ist, hergestellt mittels eines Verfahrens gemäß einem der vorhergehenden Ansprüche, zu einem pharmazeutisch verträglichen Träger oder Verdünnungsmittel umfaßt.

**Patentansprüche für folgenden Vertragsstaat : GR**

1. Platin(IV)-Koordinationsverbindung der allgemeinen Formel I,

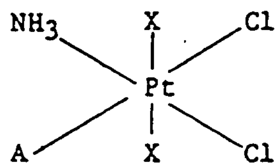

(I)

wobei

X ein Chlor-Atom oder eine Hydroxyl-Gruppe, und

A ein Amin der allgemeinen Formel R-$NH_2$ sind,

wobei

R eine geradkettige Alkyl-Kette aus 2 oder 3 Kohlenstoff-Atomen, eine alicyclische Gruppe aus 3 - 7 Kohlenstoff-Atomen oder eine verzweigte Alkyl-Kette aus 3 oder 4 Kohlenstoff-Atomen ist, mit der Maßgabe, daß R keine Cyclohexyl-Gruppe ist, wenn X Chlor ist.

2. Die Verbindung nach Anspruch 1, wobei X Chlor und A ein Amin einer verzweigten Alkyl-Kette mit 3 oder 4 Kohlenstoff-Atomen oder ein Amin einer alicyclischen Gruppe mit 3 - 5 oder 7 Kohlenstoff-Atomen sind.

3. Die Verbindung nach Anspruch 1, wobei X Hydroxyl und A ein Amin einer verzweigten Alkyl-Kette mit 3 oder 4 Kohlenstoff-Atomen oder ein Amin einer alicyclischen Gruppe mit 3 - 7 Kohlenstoff-Atomen sind.

4. Verfahren zur Herstellung einer Verbindung gemäß einem der Ansprüche 1 - 3, wobei das Verfahren den Schritt umfaßt, einen Platin(II)-Komplex der allgemeinen Formel II

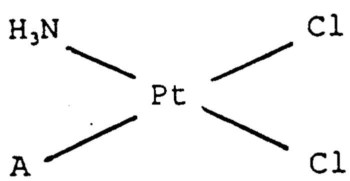

(II)

wobei A wie in Anspruch 1 definiert ist, entweder unter Bildung einer Verbindung der Formel I, wie sie in Anspruch 1 definiert ist, wobei X ein Chlor-Atom ist, mit Chlor oder unter Bildung einer Verbindung der Formel I, bei der X eine Hydroxyl-Gruppe ist, mit Wasserstoffperoxid umzusetzen.

5. Das Verfahren nach Anspruch 4, wobei der Komplex (II) in Wasser suspendiert wird, und Chlorgas durch die Suspension geleitet wird, das Reaktionsgemisch zur Entfernung überschüssigen Chlors, falls nötig, gekocht wird, und der Tetrachlor-Komplex mittels Filtration gesammelt wird.

6. Das Verfahren nach Anspruch 4, wobei der Komplex (II) in Wasser suspendiert wird, eine Wasserstoffperoxid-Lösung der Suspension unter Rühren zugesetzt wird, und das Produkt mittels Filtration gesammelt wird.

7. Das Verfahren nach einem der vorhergehenden Ansprüche, wobei der Komplex (II) dadurch hergestellt wird, daß $K[PtCl_3(NH_3)]$ durch Lösen in Wasser und Zugabe von Kaliumiodid und dem geeigneten Alkylamin in den geeigneten Alkylamin-Amin-Diiod-Komplex umgewandelt, der Diiod-Komplex mit Silbernitrat behandelt und konzentrierte Salzsäure im Überfluß zugesetzt wird.

8. Verfahren für die Herstellung einer pharmazeutischen Zusammensetzung, wobei das Verfahren die Zugabe einer Verbindung der Formel I, wie sie in Anspruch 1 definiert ist, zu einem pharmazeutisch verträglichen Träger oder Verdünnungsmittel umfaßt.

**Revendications**

**Revendications pour les Etats contractants suivants : AT, BE, CH, DE, FR, GB, IT, LI, LU, NL, SE**

**1.** Composé de coordination de platine (IV) de formule générale I,

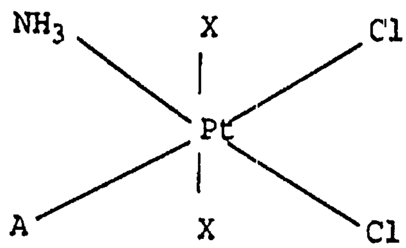

(I)

dans laquelle X est un atome de chlore ou un groupe hydroxyle, et

A est une amine de formule générale $R\text{-}NH_2$ où

R est un alkyle à chaîne droite de 2 ou 3 atomes de carbone,

alicyclique de 3 à 7 atomes de carbone, ou un alkyle à chaîne ramifiée de 3 ou 4 atomes de carbone, à condition que

si X est un atome de chlore, R ne soit pas un cyclohexyle.

**2.** Composé selon la revendication 1, caractérisé en ce que X est du chlore et A est un alkylamine à chaîne ramifiée ayant 3 ou 4 atomes de carbone ou une amine alicyclique ayant de 3 à 5 ou 7 atomes de carbone.

**3.** Composé selon la revendication 1, caractérisé en ce que X est un hydroxyle et A est une alkylamine à chaîne ramifiée avec 3 ou 4 atomes de carbone, ou une amine alicyclique avec 3 à 7 atomes de carbone.

**4.** Composition pharmaceutique comprenant un composé de platine selon l'une quelconque des revendications 1 à 3, en association avec un support ou diluant acceptable du point de vue pharmaceutique.

**5.** Composition pharmaceutique comprenant un composé de platine selon l'une quelconque des revendications 1 à 3, sous forme de dosage unitaire, comprenant une dose unitaire effective du composé de platine en association avec un support ou diluant acceptable du point de vue pharmaceutique.

**6.** Composition pharmaceutique selon la revendication 4, pour le traitement de tumeurs résistant au traitement par des composés de coordination de platine de structure différente.

**7.** Procédé de production d'un composé selon l'une quelconque des revendications 1 à 3, comprenant a) une réaction d'un complexe de platine (II) de formule générale II,

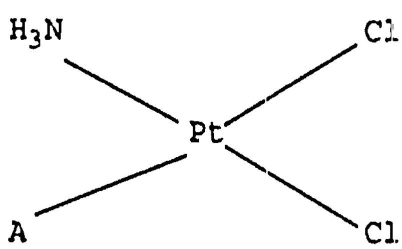

(II)

dans laquelle A est défini comme dans la revendication 1, avec du clore pour former un composé de formule I comme défini dans la revendication 1 mais dans lequel X est un atome de chlore, et b) un traitement du composé dichloré de formule II par du peroxyde d'hydrogène là où un composé de

formule I, dans lequel il y a un groupe hydroxyle, est désiré.

**Revendications pour l'Etat contractant suivant : ES**

1. Procédé de production d'un composé de coordination de platine (IV) de formule générale I,

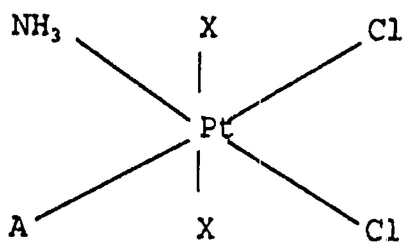

(I)

dans laquelle X est un atome de chlore ou un groupe hydroxyle, et
A est une amine de formule générale $R-NH_2$ où
R est un alkyle à chaîne droite de 2 ou 3 atomes de carbone,
alicyclique de 3 à 7 atomes de carbone, ou un alkyle à chaîne ramifiée de 3 ou 4 atomes de carbone, à condition que
si X est un atome de chlore, R ne soit pas un cyclohexyle. le procédé comportant une réaction d'un complexe de platine (II) de formule générale (II),

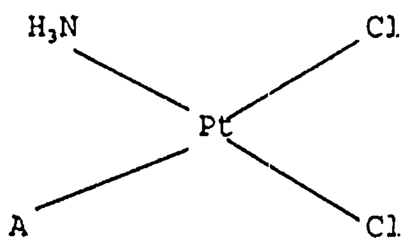

(II)

dans laquelle A est défini somme précédemment, soit avec du clore pour former un composé de formule I dans lequel X est un atome de chlore, soit du peroxyde d'hydrogène pour former un composé de formule I dans laquelle X est un groupe hydroxyde.

2. Procédé selon la revendication I, dans lequel le complexe (II) a réagi avec du chlore, caractérisé en ce que A est une alkylamine ramifiée avec 3 ou 4 atomes de carbone ou une amine alicylique de 3 à 5 ou 7 atomes de carbone.

3. Procédé selon la revendication 1, dans lequel le complexe (II) a réagi avec du peroxyde d'hydrogène, caractérisé en ce que A est une alkylamine ramifiée de 3 ou 4 atomes de carbone ou une amine alicyclique de 3 à 7 atomes de carbone.

4. Procédé selon les revendications 1 ou 2, dans lequel le complexe (II) est en suspension dans de l'eau et on fait passer du chlore gazeux à travers la suspension, le mélange de réaction est porté à l'ébullition si nécessaire pour éliminer l'excès de chlore, et le complexe tétrachloré est recueilli par filtration.

5. Procédé selon les revendications 1 ou 3, dans lequel le complexe (II) est en suspension dans de l'eau, une solution de peroxyde d'hydrogène est ajoutée à la suspension en agitant, et le produit est collecté par filtration.

6. Procédé selon l'une quelconque des revendications précédentes, dans lequel le complexe (II) est préparé par conversion de $K[PtCl_3(NH_3)]$ dans le complexe approprié diiodo amine alkylamine par

dissolution dans l'eau et addition d'iodure de potassium et de l'alkylamine appropriée, en traitant le complexe diiodo avec du nitrate d'argent et en ajoutant de l'acide chlorhydrique concentré en excès.

**7.** Procédé pour préparer une composition pharmaceutique, le procédé comprenant l'addition d'un composé de formule (I) comme défini dans la revendication 1, s'il est préparé par un procédé selon l'une quelconque revendication précédente, à un support ou diluant acceptable du point de vue pharmaceutique.

**Revendications pour l'Etat contractant suivant : GR**

**1.** Composé de coordination de platine (IV) de formule générale I,

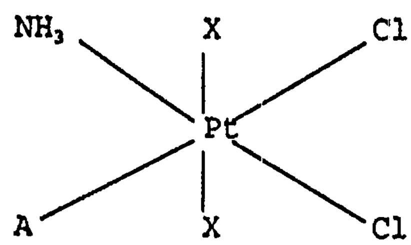

(I)

dans laquelle X est un atome de chlore ou un groupe hydroxyle, et
A est une amine de formule générale $R\text{-}NH_2$ où
R est un alkyle à chaîne droite de 2 ou 3 atomes de carbone,
alicyclique de 3 à 7 atomes de carbone, ou un alkyle à chaîne ramifiée de 3 ou 4 atomes de carbone, à condition que
si X est un atome de chlore, R ne soit pas un cyclohexyle.

**2.** Composé selon la revendication 1, caractérisé en ce que X est du chlore et A est une alkylamine à chaîne ramifiée de 3 ou 4 atomes de carbone ou une amine alicyclique de 3 à 5 ou 7 atomes de carbone.

**3.** Composé selon la revendication 1, caractérisé en ce que X est de l'hydroxyde et A est une alkylamine à chaîne ramifiée de 3 ou 4 atomes de carbone ou une amine alicyclique de 3 à 7 atomes de carbone.

**4.** Procédé de production d'un composé selon l'une quelconque des revendications 1 à 3, comportant la réaction d'un complexe de platine (II) de formule générale II

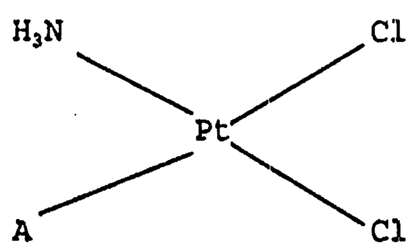

(II)

dans laquelle A est défini à la revendication 1, soit avec du clore pour former un composé de formule I comme défini à la revendication 1, dans lequel X est un atome de chlore, soit du peroxyde d'hydrogène pour former un composé de formule I dans laquelle X est un groupe hydroxyle.

**5.** Procédé selon la revendication 4, dans lequel le complexe (II) est mis en suspension dans l'eau et du chlore gazeux est envoyé à travers la suspension, le mélange de réaction est porté à l'ébullition si nécessaire pour éliminer le chlore en excès, et le complexe tétrachloro est recueilli par filtration.

**6.** Procédé selon la revendication 4, dans lequel le complexe (II) est mis en suspension dans l'eau, une solution de peroxyde d'hydrogène est ajoutée à la suspension en agitant, et le produit est recueilli par filtration.

**7.** Procédé selon l'une des revendications précédents, dans lequel le complexe (II) est préparé par conversion de $K[PtCl_3(NH_3)]$ en le complexe diiodo amine alkylamine approprié par dissolution dans l'eau, et traitement du complexe diiodo par du nitrate d'argent avec addition d'acide chlorhydrique concentré en excès.

**8.** Procédé de préparation d'une composition pharmaceutique, le procédé comprenant l'addition d'un composé de formule (I) comme défini à la revendication 1, à un support ou diluant acceptable du point de vue pharmaceutique.